Europäisches Patentamt

European Patent Office

Office européen des brevets

(19)

(11) **EP 1 067 383 B1**

(12) **EUROPÄISCHE PATENTSCHRIFT**

(45) Veröffentlichungstag und Bekanntmachung des Hinweises auf die Patenterteilung:
**02.01.2002 Patentblatt 2002/01**

(51) Int Cl.[7]: **G01N 33/22**, G01N 33/00

(21) Anmeldenummer: **99109994.6**

(22) Anmeldetag: **21.05.1999**

(54) **Verfahren zum Betrieb einer Mischvorrichtung für Gase, insbesondere zur Bestimmung des Wobbe-Indizes eines Untersuchungsgases**

Method for operating a mixing apparatus for gases, especially for determining the Wobbe index of a test gas

Méthode d'opération d'un appareil mélangeur de gaz, en particulier pour déterminer l'indice de Wobbe d'un gaz à tester

(84) Benannte Vertragsstaaten:
**DE FR GB NL**

(43) Veröffentlichungstag der Anmeldung:
**10.01.2001 Patentblatt 2001/02**

(73) Patentinhaber: **AMS Analysen, Mess-und Systemtechnik GmbH**
**69234 Dielheim (DE)**

(72) Erfinder:
• **Grienauer, Heinrich Dipl.Ing Dr.**
**76337 Waldbronn (DE)**

• **Sarpaczki, Eugen Dipl.Ing.**
**74889 Sinsheim (DE)**

(74) Vertreter: **Nunnenkamp, Jörg, Dr. et al**
**Andrejewski, Honke & Sozien Patentanwälte**
**Theaterplatz 3**
**45127 Essen (DE)**

(56) Entgegenhaltungen:
**EP-A- 0 022 493**     **EP-A- 0 323 658**
**EP-A- 0 405 693**     **EP-A- 0 445 861**
**EP-A- 0 628 815**     **US-A- 4 175 919**
**US-A- 5 486 107**

**Beschreibung**

Die Erfindung betrifft ein Verfahren zum Betrieb einer Mischvorrichtung für Gase, insbesondere zum Mischen eines Untersuchungsgases mit einem Oxidationsgas im Zuge der Bestimmung des Wobbe-Indizes des Untersuchungsgases, wobei die Mischvorrichtung mit einer Mischkammer, ferner mit zumindest einer jeweils in die Mischkammer führenden Untersuchungsgaszuführung und einer Oxidationsgaszuführung, und mit wenigstens einer Untersuchungsgasöffnung und einer Oxidationsgasöffnung in der Mischkammer ausgerüstet ist, und wobei das Untersuchungsgas und das Oxidationsgas mittels der jeweiligen Gaszuführungen über die zugehörigen Gasöffnungen in die Mischkammer zugeführt werden.

Ein Verfahren der eingangs beschriebenen Ausführungsart ist durch die EP-A 0 445 861 oder die EP-A 0 628 815 bekannt geworden. Vergleichbare Arbeitsverfahren sind Gegenstand der EP-A 0 405 693. - Damals standen Erdgase unterschiedlicher Herkunft und Qualität im Vordergrund des Interesses. Bei der nunmehr zu untersuchenden Gasen handelt es sich regelmäßig um komplexe(re) Gasgemische, insbesondere Kokerei- und/oder Raffineriegase.

Unter dem Wobbe-Index bzw. der Wobe-Zahl (WI) wird bekanntermaßen eine wichtige Kenngröße eines Gases bzw. Gasgemisches verstanden, welche sich aus dem Quotienten aus Verbrennungswärme H und Wurzel aus dem auf Luft unter Normbedingungen bezogenen Dichteverhältnis d berechnet (Einheit: MJ/m$^3$; Megajoule pro Norm-Kubikmeter oder Kilowattstunden pro N-m$^3$; d ist dimensionslos)

$$WI = {}^H/_{\sqrt{d}}$$

Insofern wird auf das deutsche Gebrauchsmuster 297 15 633 Bezug genommen.

Zur Bestimmung der Wobbe-Zahl wird in der Regel das Untersuchungsgas mit dem Oxidationsgas, z. B. Luft, vermischt und dann verbrannt, und zwar so, daß eine vollständige Verbrennung stattfindet. Nach der Verbrennung wird entweder der Temperaturanstieg oder der Restsauerstoffgehalt im Verbrennungsgas bestimmt.

Bei der erstgenannten Alternative wird die Temperaturerhöhung (und damit die Verbrennungswärme H) an einer geeigneten Stelle über der Flamme in der Reaktionskammer gemessen. - Im letztgenannten Fall wird der Verbrauch an Luftsauerstoff für die vollständige Verbrennung mittels einer Lambda-Sonde ermittelt (vgl. insgesamt in diesem Zusammenhang auch die EP-A 0 445 861, EP-A 0 405 693 und EP-A 323 658). Weitere Einzelheiten werden in dem bereits genannten deutschen Gebrauchsmuster 297 15 633 beschrieben.

Bei der Bestimmung der Wobbe-Zahl kommt es insbesondere darauf an, daß die Mischung des Untersuchungsgases bzw. Brenngases mit dem Oxidationsgas bzw. Luft konstant gehalten wird und möglichst reproduzierbar eingestellt werden kann. Denn ansonsten ist in einem nachgeschalteten Brenner oder katalytischen Reaktor (wie in DE-GM 297 15 633 beschrieben) eine definierte Verbrennung und damit beispielsweise Bestimmung des Luftbedarfs zur Ermittlung des Wobbe-Indizes nicht möglich. Insbesondere Temperatur- und Druckschwankungen führen zu Fehlern beim Mischen.

Zu diesem Zweck hat die den Ausgangspunkt der vorliegenden Lehre darstellende EP-A 0 628 815 bereits vorgeschlagen, eine Mischplatte mit mehreren Öffnungen auszurüsten, wobei der Durchmesser und die Anzahl der Öffnungen so gewählt werden, daß einerseits das gewünschte Mischungsverhältnis erreicht wird, andererseits die Reynoldszahl für Luft und Gas bzw. Untersuchungsgas gleich sein soll. Die vorbeschriebene bekannte Anordnung eignet sich für Erdgas bzw. andere Brenngase dann und nur dann, wenn die Variation der im Gas enthaltenen Gaskomponenten sehr gering ist. Derartige Schwankungen der Gaszusammensetzung lassen sich darauf zurückführen, daß die dahinterstehenden gaserzeugenden Prozesse, beispielsweise Kokereien, Hochhöfen, Chemieanlagen, Raffinerien etc. jedenfalls nicht so gesteuert bzw. gefahren werden können, daß das beispielsweise resultierende Kokereigas und Raffineriegas eine gleichbleibende Zusammensetzung an z. B. Wasserstoff, Kohlenmonoxid oder Methan sowie anderen Kohlenwasserstoffen aufweist.

Jedenfalls ist es bei derartigen Gasen, die zunehmend aus Gründen des Umweltschutzes oder als kostengünstige Alternative zum Erdgas bei der Verbrennung (zur Wärme- und/oder Stromerzeugung) Verwendung finden, unbedingt erforderlich, die dem Brenner zugeführte Energie - charakterisiert durch die Wobbe-Zahl des Brenngases und die Menge des Gases - kontinuierlich zu bestimmen, damit gleichsam der Brennwert konstant eingestellt werden kann, um eine problemlose Weiterverarbeitung zu erreichen. Dabei wird im allgemeinen so vorgegangen, dass zur Konstanthaltung der Energiezufuhr (z. B. für eine Kesselfeuerung) oder der Erreichung eines bestimmten Wertes der Energiezufuhr den gemessenen Gasen bzw. Gasgemischen definierten Mengen qualitativ hochwertigen Gases (z. B. Propan Butan usw.) zugemischt werden. Ist das Gas zu reichhaltig - was bei Raffinerie-Gasen vorkommen kann - dann wird gegebenenfalls auch mit $Co_2$ oder $N_2$ verdünnt.

Jedenfalls ist es in Anbetracht der starken Schwankungen der Konzentrationen der einzelnen Gaskomponenten schwierig, eine Konstanz des Brennwertes zu erreichen. Hierfür reicht es jedenfalls nicht aus, entsprechend der Lehre nach EP-A 0 628 815 bestimmte geometrische Verhältnisse einzustellen und dadurch die Reynoldszahl von

**EP 1 067 383 B1**

einerseits Untersuchungsgas, andererseits Oxidationsgas im Wesentlichen gleich zu bemessen.

Durch die EP-A 0 022 493 ist schließlich noch ein Verfahren und eine Vorrichtung zur verbrennungslosen Messung und/oder Regelung der Wärmemengenzufuhr zu Gasverbrauchseinrichtungen bekannt geworden. In diesem Zusammenhang wird das Brenngas bzw. ein Teilstrom des Brenngases durch mindestens einen Strömungswiderstand, der eine laminare Strömung und einen Druckanfall bewirkt, geleitet.

Der Erfindung liegt das technische Problem zugrunde, ein Verfahren zum Betrieb einer Mischvorrichtung für Gase, insbesondere zum Mischen eines Untersuchungsgases mit einem Oxidationsgas im Zuge der Bestimmung der Wobbe-Zahl des Untersuchungsgases anzugeben, welches selbst bei Gasgemischen mit Variation der Konzentration einzelner Gas-komponenten eine möglichst konstante und reproduzierbare Einstellung der Mischung und Verbrennung des Untersuchungsgases mit dem Oxidationsgas und folglich eine zuverlässige Messung der Wobbe-Zahl ermöglicht. Außerdem soll eine hierfür besonders geeignete Mischvorrichtung angegeben werden.

Zur Lösung dieser Aufgabe schlägt die Erfindung bei einem gattungsgemäßen Verfahren zum Betrieb einer Mischvorrichtung für Gase vor, dass die Untersuchungsgasöffnung und gegebenenfalls die Oxidationsgasöffnung in der Mischkammer jeweils so dimensioniert werden, dass das Untersuchungsgas und gegebenenfalls das Oxidationsgas laminar - und nicht turbulent - in die Mischkammer strömen, wobei die Temperatur und der Druck des Untersuchungsgases und des Oxidationsgases innerhalb vorgegebener Temperatur-/Druckgrenzen gleich eingestellt und konstant gehalten werden.

Selbstverständlich kann es sich auch bei dem Oxidationsgas um ein Gasgemisch handeln. Jedenfalls ist durch die laminare Strömung gewährleistet, dass innere Reibungen des Untersuchungsgases keine oder eine vernachlässigbare Rolle spielen, so dass der Durchfluss des zu messenden Gases neben seinem Druck und seiner Temperatur nur von der Dichte dieses Gases abhängt. Gleiches gilt vorzugsweise auch für das Oxidationsgas. Mit anderen Worten lässt sich durch diese erfindungsgemäße Maßnahme erreiche, dass das in die Mischkammer strömende Untersuchungsgas (wie das Oxidationsgas) dem Gesetz von Bernoulli folgen, da durch innere Reibung innerhalb des Gases bedingte Modifizierungen der vorgenannten Gesetzmäßigkeit nicht zum Tragen kommen. Dies wird mit Bezug auf die Figurenbeschreibung noch näher erläutert.

Vorzugsweise wird die Strömung so eingestellt, dass Reynoldszahlen unterhalb von 4000, vorzugsweise im Bereich von ca. 2000 und auch darunter, eingestellt werden, und zwar in der Region der Untersuchungsgasöffnung, wo die beschriebenen Probleme der inneren Reibung primär auftreten. Auch im Bereich der Oxidationsöffnung wird im Allgemeinen mit den vorgenannten Reynoldszahlen gearbeitet. Im Übrigen empfiehlt es sich, die jeweiligen Reynoldszahlen gleich einzustellen. Jedenfalls gelingt es durch eine solche Bemessungsregal, dass sich die Verhältnisse in die Mischkammer zuverlässig anhand des Durchflusses, des Drucks und der Temperatur (des Untersuchungs- und gegebenenfalls des Oxidationsgases) beschreiben lassen, so dass das entstehende Mischgas aus Untersuchungsgas und Oxidationsgas definiert und reproduzierbar eingestellt werden kann. Dies ist Grundvoraussetzung für eine zuverlässige Messung der Wobbe-Zahl des Untersuchungsgases.

Da sowohl die Temperatur als auch der Druck des Untersuchungsgases und des Oxidationsgases innerhalb vorgegebener Temperaturgrenzen bzw. Druckgrenzen jeweils gleich eingestellt und konstant gehalten werden, kann - bei gleichzeitiger Einrichtung einer laminaren Strömung im Bereich der Untersuchungsgasöffnung (und der Oxidationsgasöffnung) - die Zusammensetzung des Mischgases punktgenau voreingestellt werden, und zwar anhand der jeweiligen Durchflussraten. Darüber hinaus sieht die Erfindung vorzugsweise vor, dass das in der Mischkammer gebildete Mischgas einer als Flammensperre ausgeführten (zusätzlichen) Mischdüse zugeführt wird, damit eine vollständige Durchmischung erfolgt. Diese Mischdüse ist regelmäßig kopfseitig der Mischkammer in einem eigenen Gehäusebereich angeordnet. Au diesem Gehäusebereich gelangt das nach dem beschriebenen Verfahren erzeugte Mischgas zum Brenner oder katalytischen Reaktor, damit hier die Messung der Wobbe-Zahl erfolgen kann.

Gegenstand der Erfindung ist auch eine Vorrichtung, wie sie im geltenden Patentanspruch 6 beschrieben wird. Vorteilhafte Ausgestaltungen dieser Vorrichtung sind Gegenstand der Patentansprüche 7 bis 10.

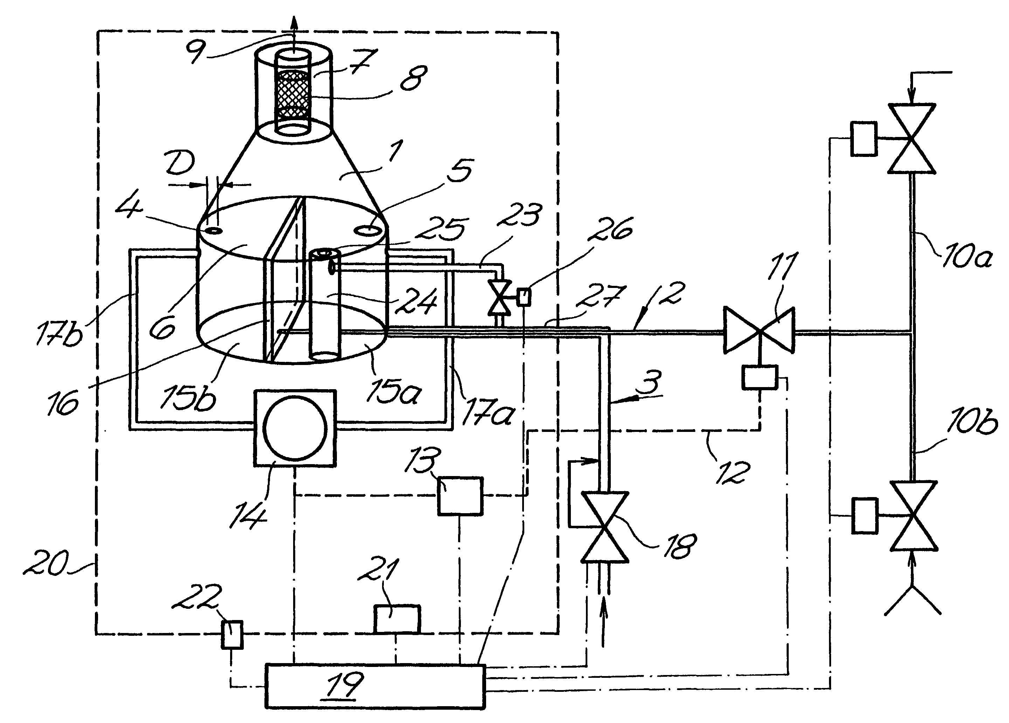

Im folgenden wird die Erfindung anhand einer lediglich ein Ausführungsbeispiel darstellenden Zeichnung näher erläutert. Die einzige Figur zeigt eine Mischvorrichtung für Gase, insbesondere zum Mischen eines Untersuchungsgases mit einem Oxidationsgas im Zuge der Bestimmung der Wobbe-Zahl des Untersuchungsgases.

Die in der zeichnerischen Darstellung zu erkennende Mischvorrichtung weist in ihrem grundsätzlichen Aufbau eine Mischkammer 1, zumindest eine in die Mischkammer 1 führende Untersuchungsgaszuführung 2 und eine Oxidationsgaszuführung 3 auf. Ferner finden sich eine Untersuchungsgasöffnung 4 und eine Oxidationsgasöffnung 5 in der Mischkammer 1. Nach dem Ausführungsbeispiel handelt es sich bei der Untersuchungsgasöffnung 4 und der Oxidationsgasöffnung 5 um Kreisöffnungen in einem bodenseitigen Abschlußblech 6 der Mischkammer 1, die ihrerseits einen kegelstumpfförmigen Außenmantel aufweist und mit einer oberseitigen Anschlußkammer 7 ausgerüstet ist. In dieser Anschlußkammer 7 befindet sich eine als Flammensperre ausgeführte Mischdüse 8. Die Flammensperre bzw. Mischdüse 8 sorgt dafür, daß ein gelegentliches Rückschlagen der nachgeschalteten Verbrennung in die Mischkammer 1 vermieden wird. - Mischkammer 1 und Anschlußkammer 7 sind aus Edelstahl gefertigt.

Von der Anschlußkammer 7 gelangt das in der Mischkammer 1 hergestellte Mischgas über eine Gasleitung

**3**

9 zu einem nicht ausdrücklich dargestellten Wobbe-Index-Meßgerät, beispielsweise einem katalytischen Reaktor, wie er in DE-GM 297 15 633 beschrieben ist. Alternativ kann an dieser Stelle auch ein Brenner vorgesehen sein, wie er grundsätzlich in den europäischen Schriften 0 323 658, 0 405 693 und 0 445 861 offenbart wird. Die Ermittlung der Wobbe-Zahl erfolgt über die Erfassung des Sauerstoffgehaltes, wie dies bereits einleitend dokumentiert wurde.

Bei dem über die Untersuchungsgaszuführung 2 zugeführten Gas handelt es sich entweder um das Untersuchungsgas, welches über eine zugehörige Gasleitung 10a in die Mischkammer 1 gelangt oder ein oder mehrere Kalibriergase, welche über eine Gasleitung 10b zugeführt werden. Zugehörige Magnetventile in den vorgenannten Leitungen bzw. Gasleitungen 10a, 10b sorgen für die gewünschte Beaufschlagung.

Im Anschluß an die vorgenannten Leitungen 10a, 10b findet sich ein Druckregler 11 innerhalb der Untersuchungsgaszuführung 2. Dieser Druckregler 11 ist über eine Druckregelleitung 12 und einen Differenzdruckregeler 13 an einen Differenz-Druck-Meßumformer 14 angeschlossen.

Die dargestellte Mischvorrichtung weist unterhalb der Mischkammer 1 eine zweigeteilte Zylinderkammer 15a, 15b auf, die kopfseitig den Boden 6 der Mischkammer 1 trägt. Dabei steht der eine Teil 15a der Zylinderkammer 15a, 15b mit der Oxidationsgaszuführung 3 in Verbindung, während die Untersuchungsgaszuführung 2 den anderen Teil 15b beaufschlagt. Beide vorgenannten Teile 15a, 15b sind durch ein Vertikalschott 16 mit zwei Schottwänden getrennt. Darüber hinaus finden sich Verbindungsleitungen 17a, 17b, die einerseits vom Zylinderkammerteil 15a zum Differenz-Druck-Meßumformer 14, andererseits vom Zylinderkammerteil 15b zum Differenz-Druck-Meßumformer 14 führen. Folglich läßt sich mittels des Differenz-Druck-Meßumformers 14 ein Druckunterschied zwischen den beiden vorgenannten Zylinderkammern 15a, 15b erfassen. Je nachdem, ob ein Druckunterschied vorliegt, wird gegebenenfalls (zusätzliches) Untersuchungsgas über die Druckregelleitung 12 in die Zylinderkammer 15b zugeführt.

Dabei ist es gleichzeitig möglich, einen zusätzlichen Druckregler 18 in der Oxidationsgaszuführung 3 entsprechend zu beaufschlagen. Dies alles geschieht mittels einer Steuer-/Regeleinrichtung 19. Diese wertet nicht nur Meßdaten des Differenz-Druck-Meßumformers 14 aus und steuert den Differenzdruckregler 13 entsprechend, sondern dient auch zur Betätigung der Druckregler 11 und 18. Ebenso können die Magnetventile in den Leitungen 10a, 10b von der Steuer-/Regeleinrichtung 19 beaufschlagt werden. Folglich kann der Druck in beiden Zylinderkammern 15a, 15b einerseits mittels des Druckreglers 18, andererseits mittels des Differenzdruckreglers 13 in Verbindung mit der Druckregelleitung 12 feinfühlig und getrennt eingestellt werden, und zwar je nachdem ob ein Differenzdruck vom Differenz-Druck-Meßumformer 14 erfaßt wurde und bejahendenfalls in Abhängigkeit von dessen Größe. Dies erfolgt mittels der Steuer-/Regeleinrichung 19 im Zuge eines geschlossenen Regelkreises.

Die Temperatur des Untersuchungsgases und des Oxidationsgases wird innerhalb vorgegebener Temperaturgrenzen gleich eingestellt. Zu diesem Zweck ist die Mischkammer 1 inklusive Anschlußgehäuse 7 sowie Zylinderkammern 15a, 15b und entsprechender Anbauteile 13, 14 in einer Wärmeisolationskammer 20 angeordnet, deren Innen- und gegebenenfalls Außenwandung aus einem wärmeisolierenden Material, beispielsweise Keramik, besteht oder hiermit beschichtet ist.

Zusätzlich kann sich der nicht dargestellte Brenner bzw. der katalytische Reaktor in dieser Wärmeisolationskammer 20 befinden. Dadurch läßt sich eine gleiche Temperatur des Untersuchungsgases und des Oxidationsgases einstellen, und zwar mit einer relativen Abweichung von ca. ± 1°C bis ± 5°C, vorzugsweise ± 2°C. Zum Heizen wird die Abwärme des nicht ausdrücklich dargestellten Brenners bzw. Reaktors genutzt, während zur Kühlung ein steuerbarer Ventilator 21 vorgesehen ist, welcher mit der Außenluft in Verbindung steht. Dieser Ventilator 21 läßt sich ebenso wie ein in bzw. an der Wärmeisolationskammer vorgesehener Temperatursensor 22 von der bereits beschriebenen Steuer-/Regeleinrichtung 19 beaufschlagen, an welche die vorgenannten Bauteile 21, 22 angeschlossen sind. In Abhängigkeit von mit Hilfe des Temperatursensors 22 gemessenen Temperatur-Istwerten wird der steuerbare Ventilator 21 zur Einstellung vorgegebener Temperatur-Sollwerte beaufschlagt, und zwar im Sinne einer Regelung.

Schließlich ist eine zusätzliche Luftzuführung 23 zur Kalibrierung der Mischkammer 1 und/oder des Brenners bzw. katalytischen Reaktors vorgesehen. Diese zusätzliche Luftzuführung 23 mündet in eine eigene Kalibrierkammer 24 mit kopfseitiger Kalibrieröffnung 25 im Boden 6 der Mischkammer 1.

Mit Hilfe dieser zusätzlichen Luftzuführung 23 läßt sich bei einem bestimmten und gemessenen Wobbe-Index durch die Zufuhr zusätzlicher Luft bzw. eines Oxidationsgases (vorgegebener und definierter Menge) eine Verdünnung des in der Mischkammer 1 aufbereiteten Mischgases und folglich eine gezielte Erniedrigung der Wobbe-Zahl erreichen. Diese Erniedrigung bzw. Verdünnung erfolgt determiniert, weil die Kalibrierkammer 24 und die Kalibrierdüse 25 die Zufuhr lediglich eines bestimmten zusätzlichen Gasflusses ermöglichen. Zu diesem Zweck ist in der zusätzlichen Luftzuführung 23 gleichfalls ein Magnetventil 26 vorgesehen, welches ebenfalls von der Steuer-/Regeleinrichtung 19 beaufschlagt wird.

Um die Temperatur des Untersuchungsgases wie des Oxidationsgases möglichst gleich einzustellen, werden die Untersuchungsgaszuführung 2 und die Oxidationsgaszuführung 3 nebeneinander liegend bzw. als konzentrische Rohre zumindest im Bereich der Wärmeisolationskammer 20 geführt. Hier ist ein Wärmeaustauscher 27 realisiert, welcher für einen zuverlässigen Wärmetransport zwischen den beiden vorgenannten Gasen sorgt.

Mit Hilfe des Differenzdruckreglers 13 in Verbindung mit dem Differenz-Druck-Meßumformer 14 sowie der Steuer-/Regeleinrichtung 19, der Differenzdruckleitung 12 und schließlich des Druckreglers 18 läßt sich der Druck in der Zylinderkammer 15a im Vergleich zur Zylinderkammer 15b praktisch gleich einstellen. Jedenfalls sind geregelte Differenzdrucke zwischen Untersuchungsgas und Oxidationsgas von bis zu ca. ± 0,1 mbar möglich. In diesem Zusammenhang ist der Druck des zugeführten Oxidationsgases wie des Untersuchungsgases gleich und konstant zu halten. Dabei haben sich in der Praxis Druckschwankungen von ± 100 mbar als unschädlich erwiesen, vorausgesetzt, das Oxidationsgas wie das Untersuchungsgas sind den gleichen Schwankungen unterworfen und der Differenzdruck zwischen den beiden vorgenannten Gasen wird auf den vorgenannten Wert von ca. ± 0,1 mbar geregelt.

Im Bereich der Untersuchungsgasöffnung 4 sowie gegebenenfalls der Oxidationsgasöffnung 5 wird erfindungsgemäß dafür gesorgt, daß hier laminare Strömungsverhältnisse vorliegen. Derartiges ist grundsätzlich möglich, weil die Zylinderkammern 15a, 15b praktisch wie Druckreservoirs wirken, von denen aus sich das jeweilige Gas in die Mischkammer 1 entspannt. Mit anderen Worten treten die zu mischenden Gase in die Mischkammer 1 praktisch ohne innere Reibung ein. Hierbei macht sich die beschriebene Lehre zu Nutze, daß sich der Einfluß der inneren Reibung in einer Strömung durch eine Öffnung mit hierdurch verursachtem Druckverlust ∆p wie folgt beschreiben läßt:

$$1.1) \qquad \Delta P = [1 + f(Re)] \cdot \frac{1}{2} \cdot \rho \cdot v^2$$

Dabei bezeichnet Re die Reynoldszahl, $\rho$ die Dichte und v die Durchflußgeschwindigkeit in der Reduzierung bzw. in der zugehörigen Öffnung 4,5. Bei Reynoldszahlen unter 2000 nähert sich die vorgenannte Funktion f(Re) einem Nulldurchgang, so daß Verhältnisse erreicht werden, die dem Gesetz von Bernoulli folgen. Reynoldszahlen über 4000 charakterisieren dagegen turbulente Strömungen mit hoher innerer Reibung, die zu signifikanten Abweichungen vom Bernoulli-Gesetz

$$1.2) \qquad \Delta P = \frac{1}{2} \cdot \rho \cdot v^2$$

korrespondieren. Mit anderen Worten kommt es im Rahmen der Erfindung besonders darauf an, im Zuge der Durchmischung von Untersuchungsgas und Oxidationsgas in der Mischkammer 1 bzw. im Bereich der Reduzierung dafür zu sorgen, daß an dieser Stelle (in der Region der Öffnungen 4,5) laminare Strömungsverhältnisse vorliegen. Dies ist dann gewährleistet, wenn Reynoldszahlen um 2000 oder darunter eingestellt werden. Um derartige Werte zu erreichen, muß man berücksichtigen, daß sich die Reynoldszahl Re wie folgt bemißt:

$$1.3) \qquad Re = \frac{\rho \cdot v \cdot D}{\eta}$$

mit $\eta$ der dynamischen Viskosität (gemessen in Centi-Poise oder in Pascal-Sekunden) und D dem Durchmesser der jeweiligen Öffnung 4, 5. Diese dynamische Viskosität $\eta$ hängt von der Temperatur des Gases gemäß der folgenden empirischen Gesetzmäßigkeit ab:

$$1.4) \qquad \eta = 10^{-3} * K * (10^{-4} * T)^c$$

mit K und c verschiedenen Stoffkonstanten, die in der nachfolgenden Tabelle für einzelne Gas-Komponenten aufgeführt sind:

| Gas-Komponente | K | c |
|---|---|---|
| Luft | 0,194 | 0,674 |
| Methan | 0,185 | 0,803 |
| Kohlendioxid | 0,298 | 0,854 |
| Kohlenmonoxid | 0,194 | 0,674 |
| Wasserstoff | 0,089 | 0,652 |
| Ethan | 0,225 | 0,907 |
| Propan | 0,153 | 0,829 |
| Butan | 0,200 | 0,935 |

(fortgesetzt)

| Gas-Komponente | K | c |
|---|---|---|
| Pentan | 0,467 | 1,230 |
| Ethylen | 0,206 | 0,854 |

Folglich lassen sich anhand der erwarteten oder in einer vorgeschalteten und nicht dargestellten Gasanalyse-Einrichtung gemessenen und mit Hilfe der Steuer-/Regeleinrichtung 19 ausgewerteten Variation der Konzentration einzelner Gaskomponenten des Untersuchungsgases hierzu korrespondierende Schwankungen der dynamischen Viskosität $\eta$ des Untersuchungsgases anhand der Gleichung 1.4) ableiten. Daraus können Variationen der Reynoldszahl gemäß der Vorschrift 1.3) ermittelt werden, so daß schließlich die gewünschte Einstellung der Reynoldszahl im Bereich von ca. 2000 oder darunter gelingt, und zwar durch entsprechende Bemessung des Durchmessers D der Reduzierung bzw. der zugehörigen Öffnung 4, 5, wenn gleichzeitig v gemessen oder vorgegeben wird.

Dabei kann der vorgenannte Durchmesser D entweder fest eingestellt werden, und zwar je nach den erwarteten Schwankungen der dynamischen Viskosität $\eta$ oder auch variabel, indem beispielsweise eine irisartige Öffnung 4,5 mit veränderbarem Durchmesser D zum Einsatz kommt. Dieser Durchmesser D kann von der Steuer-/Regeleinrichtung 19 variiert werden, und zwar je nachdem welches Gasgemisch untersucht werden soll bzw. vorliegt.

Die folgende Tabelle zeigt nur beispielhaft, wie die einzelnen Gaskomponenten eines Kokerei- sowie eines Raffineriegases schwanken können:

| | Kokereigas | | Raffineriegas | |
|---|---|---|---|---|
| | Minimum Vol% | Maximum Vol% | Minimum Vol% | Maximum Vol% |
| Wasserstoff $H_2$ | 58,3 | 70,2 | 37,0 | 83,5 |
| Kohlenmonoxid CO | 4,0 | 6,2 | 2,7 | 20,3 |
| Methan CH4 | 19,5 | 25,5 | 3,4 | 18,9 |
| Ethan C2H6 | 0,4 | 0,9 | 3,3 | 11,7 |
| Ethen C2H4 | 1,7 | 2,4 | 2,0 | 5,2 |
| Ethylen C2H2 | | | 0,0 | 4,9 |
| Propan C3H8 | | | 3,7 | 12,3 |
| Butan C4H10 | | | 1,6 | 3,5 |
| $\Sigma$ Höhere Kohlenwasserstoffe | | | 0,3 | 4,8 |
| | | | | |
| | | | | |
| Schwefelwasserstoff H2S | 0,00 | 4,10 | 0,0 | 0,1 |
| | | | | |
| Sauerstoff | 0,20 | 0,70 | 0,0 | 0,1 |
| Kohlendioxid | 1,40 | 1,90 | 0,6 | 26,3 |
| Stickstoff | 2,90 | 6,10 | 0,1 | 5,9 |

Selbstverständlich liegt es im Rahmen der Erfindung, mit einem entsprechenden Applikationsprogramm in der Steuer-/Regeleinrichung 19 zu arbeiten, welches aus den Konzentrationen der Gaskomponenten des Untersuchungsgases die zu erwartende Reynoldszahl Re berechnet und den Durchmesser D der korrespondierenden Öffnung 4 entsprechend einstellt. Gleiches ist generell für die Oxidationsgasöffnung 5 denkbar, jedoch zumeist nicht erforderlich, weil hier das Problem der inneren Reibung durch verschiedene Gas-komponenten bei gegebenen Gasfluß in der Regel keine Rolle spielt, es sei denn, das Oxidationsgas besteht auch aus verschiedenen Gaskomponenten.

**Patentansprüche**

1. Verfahren zum Betrieb einer Mischvorrichtung für Gase, insbesondere zum Mischen eines Untersuchungsgases mit einem Oxidationsgas im Zuge der Bestimmung der Wobbe-Zahl des Untersuchungsgases, wobei die Mischvorrichtung mit

   - einer Mischkammer (1), und mit

   - zumindest einer jeweils in die Mischkammer (1) führenden Untersuchungsgaszuführung (2) und einer Oxidationsgaszuführung (3), ausgerüstet ist,

   und wobei das Untersuchungsgas und das Oxidationsgas in die Mischkammer (1) zugeführt werden, **dadurch gekennzeichnet, dass** das Untersuchungsgas laminar in die Mischkammer einströmt, wobei die Temperatur und der Druck des Untersuchungsgases und des Oxidationsgases innerhalb vorgegebener Temperatur-/Druckgrenzen gleich eingestellt und konstant gehalten werden.

2. Verfahren nach Anspruch 1, **dadurch gekennzeichnet, dass** zumindest im Bereich einer Untersuchungsgasöffnung (4) Reynoldszahlen unterhalb von 4000, vorzugsweise im Bereich von ca. 2000 und auch darunter, eingestellt werden.

3. Verfahren nach Anspruch 1 oder 2, **dadurch gekennzeichnet, dass** die Temperatur des Untersuchungsgases und des Oxidationsgases im Sinne einer Regelung eingestellt werden.

4. Verfahren nach einem der Ansprüche 1 bis 3, **dadurch gekennzeichnet, dass** der Druck des Untersuchungsgases und des Oxidationsgases im Sinne einer Regelung eingestellt werden.

5. Verfahren nach einem der Ansprüche 1 bis 4, **dadurch gekennzeichnet, dass** das in der Mischkammer (1) gebildete Mischgas einer als Flammensperre ausgeführte, kopfseitig der Mischkammer (1) angeordneten, Mischdüse (8) zugeführt wird.

6. Mischvorrichtung für Gase, insbesondere zum Mischen eines Untersuchungsgases mit einem Oxidationsgas im Zuge der Bestimmung der Wobbe-Zahl des Untersuchungsgases zur Durchführung des Verfahrens nach einem der Ansprüche 1 bis 5, mit

   - einer Mischkammer (1), ferner mit

   - zumindest einer jeweils in die Mischkammer (1) führenden Untersuchungsgaszuführung (2) und einer Oxidationsgaszuführung (3), und mit

   - wenigstens einer Untersuchungsgasöffnung (4) und einer Oxidationsgasöffnung (5) in der Mischkammer (1),

   wobei das Untersuchungsgas und das Oxidationsgas mittels der jeweiligen Gaszuführungen (2,3) über die zugehörigen Gasöffnungen (4, 5) in die Mischkammer (1) zugeführt werden,
   **dadurch gekennzeichnet, dass**

   - zumindest der Durchmesser (D) der Untersuchungsgasöffnung (4) so dimensioniert ist, dass das Untersuchungsgas laminar in die Mischkammer (1) strömt, wobei

   - zur Gleicheinstellung und Konstanthaltung der Temperatur des Untersuchungsgases und des Oxidationsgases die Mischkammer (1) in einer Wärmeisolationskammer (20) angeordnet ist, und wobei

   - zur Gleicheinstellung und Konstanthaltung des Druckes des Untersuchungsgases und des Oxidationsgases ein Differenz-Druck-Messumformer (14) vorgesehen ist.

7. Vorrichtung nach Anspruch 6, **dadurch gekennzeichnet, dass** die Mischkammer (1) gegebenenfalls zusammen mit einem nachgeschalteten Brenner oder einem katalytischen Reaktor zur Bestimmung der Wobbe-Zahl des Untersuchungsgases in einer Wärmeisolationskammer (20) angeordnet ist.

# EP 1 067 383 B1

8. Vorrichtung nach Anspruch 6 oder 7, **dadurch gekennzeichnet, dass** die Wärmeisolationskammer (20) mittels Abwärme des Brenners oder des katalytischen Reaktors beheizt wird und mit Hilfe eines mit Außenluft in Verbindung stehenden Ventilators (21) gekühlt wird.

9. Vorrichtung nach einem der Ansprüche 6 bis 8, **dadurch gekennzeichnet, dass** die Wärmeisolationskammer (20) zumindest einen an eine Steuer-/Regeleinrichtung (19) angeschlossenen Temperatursensor (22) aufweist, wobei die Steuer-/Regeleinrichtung (19) in Abhängigkeit von gemessenen Temperatur-Istwerten den Ventilator (21) zur Einstellung vorgegebener Temperatur-Sollwerte entsprechend beaufschlagt.

10. Vorrichtung nach einem der Ansprüche 6 bis 9, **dadurch gekennzeichnet, dass** eine zusätzliche Luftzuführung (23) zur Kalibrierung der Mischkammer (1) und/oder des Brenners oder katalytischen Reaktors vorgesehen ist.

**Claims**

1. A procedure for operating a mixing device for gasses and, in particular for mixing an analysing gas with an oxidation gas for determining the Wobbe index of the analysing gas, in which the mixing device comprises

   - a mixing chamber (1) and

   - at least one analysing gas supply line (2) leading to the mixing chamber (1) and one oxidation gas supply line (3)

   and in which the analysing gas and the oxidation gas are supplied to the mixing chamber (1), **characterised in that** the analysing gas flows steadily into the mixing chamber with the temperature and pressure of the analysing gas and of the oxidation gas being set to the same value and maintained within specified temperature/pressure limits.

2. A procedure according to claim 1, **characterised in that** at least in the area of the analysing gas opening (4), Reynolds numbers below 4000 and preferably around approx. 2000 or lower are set.

3. A procedure according to claim 1 or 2, **characterised in that** the temperature of the analysing gas and the oxidation gas is set for control purposes.

4. A procedure according to one of the claims 1 to 3, **characterised in that** the pressure of the analysing gas and the oxidation gas is set for control purposes.

5. A procedure according to one of the claims 1 to 4, **characterised in that** the mixed gas formed in the mixing chamber (1) is supplied to a mixing nozzle (8) designed as a flame arrester and arranged at the head of the mixing chamber (1).

6. A mixing device for gasses, particular for mixing an analysing gas with an oxidation gas for determining the Wobbe index of the analysing gas, for carrying out the procedure according to claims 1 to 5, comprising

   - a mixing chamber (1) and

   - at least one analysing gas supply line (2) leading to the mixing chamber (1) and one oxidation gas supply line (3) and

   - at least one analysing gas opening (4) and one oxidation gas opening (5) in the mixing chamber (1),

   in which the analysing gas and the oxidation gas are supplied to the mixing chamber (1) by the respective gas supply lines (2, 3) via the associated gas openings (4, 5),
   **characterised in that**

   - at least the diameter (D) of the analysing gas opening (4) is dimensioned in such a way that the analysing gas flows steadily into the mixing chamber (1), in which

   - the mixing chamber (1) is located in a heat insulation chamber (20) for providing an evenly set and constantly maintained temperature of the analysing gas and the oxidation gas and in which

- a differential pressure transducer (14) is provided for ensuring an evenly set and constantly maintained pressure of the analysing gas and the oxidation gas.

7.  A device according to claim 6, **characterised in that** the mixing chamber (1) and, where applicable, a downstream burner or a catalytic reactor for determining the Wobbe index are arranged in the heat insulation chamber (20).

8.  A device according to claims 6 or 7, **characterised in that** the heat insulation chamber (20) is heated with the waste heat of the burner or of the catalytic reactor and is cooled by a fan (21) in contact with external air.

9.  A device according to one of the claims 6 to 8, characterised in that the heat insulation chamber (20) comprises at least one temperature sensor (22) connected to the control/regulating unit (19), with the control/regulating unit (19) activating the fan (21) for providing the specified set temperature values, depending on measured actual temperature values.

10. A device according to one of the claims 6 to 9, **characterised in that** an additional air supply line (23) is provided for calibrating the mixing chamber (1) and/or the burner or catalytic reactor.

## Revendications

1.  Méthode de fonctionnement d'un dispositif de mixtion pour gaz, destiné notamment à mélanger un gaz d'essai avec un gaz d'oxydation lors de la détermination de l'indice de Wobbe du gaz d'essai; ce dispositif de mixtion étant équipé de

    - une chambre de mélange (1) et

    - au moins une conduite d'alimentation pour le gaz d'essai (2) et une conduite d'alimentation pour le gaz d'oxydation (3), menant respectivement à la chambre de mélange (1) ;

    le gaz d'essai et le gaz d'oxydation étant amenés dans la chambre de mélange (1); **caractérisée par le fait que** le gaz d'essai afflue dans la chambre de mélange de manière laminaire; la température et la pression du gaz d'essai et du gaz d'oxydation sont réglées d'emblée et maintenues constantes au sein de plages de température/pression prescrites.

2.  Méthode selon la revendication 1, **caractérisée par** des nombres de Reynolds inférieurs à 4000, situés de préférence aux environs de 2000 ou inférieurs, du moins dans la zone de l'ouverture pour le gaz d'essai (4).

3.  Méthode selon la revendication 1 ou 2, **caractérisée par le fait que** la température du gaz d'essai et celle du gaz d'oxydation sont réglées par un système de régulation.

4.  Méthode selon la revendication 1, 2 ou 3, **caractérisée par le fait que** la pression du gaz d'essai et celle du gaz d'oxydation sont réglées par un système de régulation.

5.  Méthode selon la revendication 1, 2, 3 ou 4, **caractérisée par le fait que** le mélange gazeux formé dans la chambre de mélange (1) est amené vers une buse de mélange (8) disposée au niveau de la tête de la chambre de mélange (1) et construite comme un dispositif anti-retour de flamme.

6.  Dispositif de mixtion pour gaz, destiné notamment à mélanger un gaz d'essai avec un gaz d'oxydation lors de la détermination de l'indice de Wobbe du gaz d'essai, destiné à mettre en oeuvre la méthode conformément à la spécification 1, 2, 3, 4 ou 5; ce dispositif de mixtion étant équipé de

    - une chambre de mélange (1) et

    - au moins une conduite d'alimentation pour le gaz d'essai (2) et une conduite d'alimentation pour le gaz d'oxydation (3), menant respectivement à la chambre de mélange (1);

EP 1 067 383 B1

- au moins une ouverture pour le gaz d'essai (4) et une ouverture pour le gaz d'oxydation (5) dans la chambre de mélange (1),

le gaz d'essai et le gaz d'oxydation étant amenés dans la chambre de mélange (1) au moyen des conduites d'alimentation respectives (2, 3) par l'intermédiaire des ouvertures correspondantes (4, 5),
**caractérisé par le fait que**

- au moins le diamètre (D) de l'ouverture pour le gaz d'essai (4) est dimensionné de sorte que le gaz d'essai afflue dans la chambre de mélange (1) de manière laminaire

- la chambre de mélange (1) étant logée dans une chambre dotée d'une isolation thermique (20) pour régler d'emblée et maintenir constante la température du gaz d'essai et celle du gaz d'oxydation,

- un convertisseur de mesure à pression différentielle (14) étant prévu pour régler d'emblée et maintenir constante la pression du gaz d'essai et celle du gaz d'oxydation.

7. Dispositif selon la revendication 6, **caractérisé par le fait que** la chambre de mélange (1) est le cas échéant logée dans une chambre dotée d'une isolation thermique (20), avec un brûleur ou un réacteur catalytique en aval pour la détermination de l'indice de Wobbe du gaz d'essai.

8. Dispositif selon la revendication 6 ou 7, **caractérisé par le fait que** la chambre dotée d'une isolation thermique (20) est chauffée au moyen de la chaleur dissipée par le brûleur ou le réacteur catalytique et refroidie au moyen d'un ventilateur (21) en liaison avec l'air extérieur.

9. Dispositif selon la revendication 6, 7 ou 8, **caractérisé par le fait que** la chambre dotée d'une isolation thermique (20) présente au moins un capteur de température (22) raccordé à un système de commande/ régulation (19), ce dernier pilotant le ventilateur (21) pour régler les valeurs de consigne de température en fonction des valeurs de température réelles mesurées.

10. Dispositif selon la revendication 6, 7, 8 ou 9, **caractérisé par le fait qu'**une conduite d'alimentation d'air supplémentaire (23) est prévue pour calibrer la chambre de mélange (1) et/ou le brûleur ou le réacteur catalytique.